# FASCICULE DE BREVET EUROPEEN

(11) **EP 3 527 238 B1**
(45) Date de publication et mention de la délivrance du brevet: **11.11.2020**
(21) Numéro de dépôt: 19157595.0
(22) Date de dépôt: 15.02.2019
(51) Int. Cl.: A61M 1/06, A61J 9/00

(54) **APPAREIL TIRE-LAIT**
BRUSTPUMPE
BREASTPUMP

(30) Priorité: 16.02.2018 FR 1800151
(43) Date de publication de la demande: 21.08.2019
(73) Titulaire: El Mahboub, Radouane, 83600 Frejus (FR)
(72) Inventeur: El Mahboub, Radouane, 83600 Frejus (FR)
(74) Mandataire: Nicolle, Olivier

(56) Documents cités:
- WO-A1-2011/144984
- WO-A1-2016/024558
- DE-C- 729 251
- US-A1- 2015 038 945

## Description

### Domaine de l'invention

La présente invention concerne un appareil tire-lait, en particulier, un appareil pour aider à exprimer du lait directement à un nourrisson de façon efficace et avec flexibilité.

### Arrière-plan technologique

L'importance de l'allaitement maternel est reconnue dans de nombreuses sociétés développées et en développement.

Certains utilisateurs, ou mères, peuvent trouver difficile d'exprimer leur lait et peuvent utiliser des moyens de pompage afin d'extraire le lait de leurs seins pour que leur bébé puisse le consommer ultérieurement.

Cependant, en plus de la valeur nutritionnelle du lait maternel, l'allaitement maternel est reconnu comme une importante étape dans l'établissement de lien affectif entre la mère et l'enfant.

De ce fait, il peut être préférable d'allaiter directement au sein, mais ceci devient problématique si le lait est insuffisant. En cas de problèmes d'expression du lait, il peut également être difficile de déterminer la quantité de lait ingérée par le nourrisson. Si cette quantité est trop faible, le nourrisson et/ou la mère peuvent ressentir de la frustration au détriment du lien affectif. Une forme d'anxiété peut alors être provoquée chez la mère par le fait que le nourrisson n'a pas suffisamment été nourri.

### État de la technique antérieure

Le document US 2011 270 164 (BANE) décrit un dispositif pour permettre l'allaitement de nourrissons à partir d'une courte distance sans les étapes impliquées dans le pompage et l'alimentation au biberon du lait maternel. Un exemple d'une telle utilisation est lorsque la mère et l'enfant montent dans une voiture et restent attachés en toute sécurité dans un siège de voiture avec une ceinture de sécurité, respectivement. Le dispositif en une ou trois pièces relie la mère au nourrisson par l'intermédiaire d'un tube flexible muni d'un protège-sein adhésif à centre ouvert pour recueillir le lait à une extrémité et d'un mamelon artificiel pour l'allaitement du nourrisson à l'autre extrémité. L'invention peut être facilement nettoyée et stérilisée pour des utilisations multiples. L'appareil peut être une unité simple ou trois pièces facilement détachables pour permettre une utilisation et un nettoyage encore plus faciles.

Le document CN 103 301 518 (LIU) concerne un compteur en temps réel pour le lait maternel aspiré par un bébé. Le compteur en temps réel comprend un composant de collecte de lait maternel, un débitmètre et un composant de succion pour le bébé qui sont connectés séquentiellement. Après que le lait maternel a été recueilli par le composant de collecte du lait maternel, le lait maternel passe dans le débitmètre et entre finalement dans le composant de succion destiné au bébé. Le compteur en temps réel présente l'avantage d'être à la fois simple dans sa structure et simple et pratique à utiliser, la quantité de lait maternel aspiré par un bébé peut être mesurée avec précision en temps réel, et le compteur temps réel a une perspective de marché large.

Le document WO 201 095 133 (HARARI et al) décrit un dispositif pour mesurer une quantité de lait maternel consommé pendant une session d'allaitement, le dispositif comprenant : un mécanisme pour déterminer un changement de volume d'un sein d'avant la session d'allaitement à après la session d'allaitement maternel ; et une unité de calcul pour calculer, à partir de ladite variation de volume, une quantité de lait maternel exprimée pendant la session d'allaitement.

On connaît par ailleurs les documents WO 2011/144984 A1, US 2015/038945, DE729251C et WO 2016/024558 A1 qui proposent différents types de tire-lait, lesquels ne sont pas satisfaisants pour les utilisatrices.

La nécessité de surmonter les problèmes rencontrés sur les dispositifs existants a conduit à la présente invention.

### Résumé de l'invention

Un appareil tire-lait selon la présente invention est défini dans la revendication indépendante 1. L'appareil tire-lait comprend: un dispositif avec un adaptateur mammaire ; et une pompe pour aspirer le fluide à partir de l'adaptateur mammaire ; ainsi qu'un bras articulé supportant la tétine et permettant une variation d'angle par rapport à l'adaptateur mammaire, dans lequel le dispositif comprend une tétine et des moyens de mesure de fluide afin de mesurer le volume de fluide à l'intérieur du dispositif.

En pratique, la tétine est disposée sur un mécanisme de support à bras formant bras articulé, s'étendant à partir du corps du dispositif où le bras est agencé pour être reconfiguré de manière à s'étendre sous différents angles depuis le corps. De cette manière, l'utilisateur peut bénéficier d'une plus grande flexibilité de mouvement et de positionnement du nourrisson pendant l'allaitement.

Ainsi, l'appareil de la présente invention permet à un utilisateur, ou une mère, d'exprimer efficacement son lait, d'être directement lié avec le nourrisson pendant l'allaitement ainsi que de réguler efficacement la quantité de lait ingérée.

L'adaptateur mammaire peut être disposé sur un mécanisme de support s'étendant depuis le corps, par exemple en formant un angle avec le corps, ou de manière à permettre une variation du positionnement de l'adaptateur par rapport au corps.

Les moyens de mesure peuvent être agencés dans certains modes de réalisation pour mesurer d'autres variables associées au fluide, telles que la température ou la vitesse d'écoulement.

Les moyens de mesure peuvent être configurés, dans certains modes de réalisation, pour mesurer l'écoulement à travers la tétine, et dans certains modes de réalisation peuvent être configurés pour mesurer l'écoulement de fluide à travers l'adaptateur mammaire.

Selon un mode alternatif de réalisation conforme à l'invention, le dispositif peut comprendre un réservoir, lequel réservoir peut permettre un stockage temporaire de fluide pendant l'alimentation ou ultérieurement. De cette manière, dans certains modes de réalisation, les moyens de mesure peuvent permettre une mesure du volume dans le réservoir et/ou du volume de fluide à partir de l'adaptateur mammaire.

Dans certains modes de réalisation, la pompe est fournie séparément du dispositif. Dans de tels modes de réalisation, la pompe peut être détachée du dispositif. La pompe peut par conséquent comprendre un moyen de pompage électrique ou un moyen de pompage manuel, ou permettre l'un ou l'autre.

L'adaptateur mammaire peut être disposé à une première extrémité du dispositif et la tétine peut être disposée à une seconde extrémité, par exemple lorsque le dispositif est un corps allongé.

Dans certains modes de réalisation, l'adaptateur mammaire comprend un embout, par exemple un embout de forme conique, ayant une base, un sommet et un espace intérieur ouvert ; dans lequel une extrémité creuse s'étend à partir du sommet et communique avec l'espace intérieur ouvert, dans lequel le mamelon peut être insérer dans l'extrémité creuse.

L'adaptateur mammaire peut être détachable du dispositif ou du corps, par exemple de manière à permettre le remplacement de tailles, de dimensions ou d'agencements différents en fonction de la taille du mamelon et/ou du sein.

Les moyens de mesure peuvent être situés à l'intérieur du corps du dispositif pour faciliter la mesure. De plus ou alternativement, un affichage visuel local peut être prévu pour les moyens de mesure qui sont situés au centre de l'appareil. Dans certains modes de réalisation, l'affichage visuel peut être déplacé du corps du dispositif pour permettre une meilleure lecture lors de l'utilisation par l'utilisateur.

L'affichage visuel peut être configuré pour se replier vers le haut par exemple. Dans d'autres modes de réalisation, les moyens de mesure peuvent, de plus ou alternativement, fournir un affichage visuel à distance, par exemple dans lequel le dispositif peut comprendre une connexion câblée ou sans fil pour l'affichage visuel. Certains modes de réalisation peuvent donc être activés sans fil, comprenant une connectivité Bluetooth, infrarouge ou par radiofréquence, qui peut permettre à l'utilisateur de lire l'affichage via un appareil électronique fourni indépendamment.

Dans certains modes de réalisation, la pompe peut être connectée en cours d'utilisation pour favoriser le passage du lait de l'adaptateur mammaire vers la tétine.

Dans certains modes de réalisation, la tétine peut être détachée, par exemple de sorte que le dispositif peut être utilisé pour exprimer du lait en vue de son stockage. Le remplacement de la tétine peut comprendre un adaptateur ou une bouteille de stockage, qui peuvent être inclus dans l'appareil de la présente invention.

En outre ou alternativement, dans certains modes de réalisation, l'appareil peut comprendre des moyens d'adaptation pour la tétine, et un bras de support de manière à permettre la formation d'un récipient de type bouteille ou biberon, qui peut comprendre un couvercle. De cette manière, l'appareil peut permettre le transport et le stockage de lait ou de fluide, sans le dispositif.

Dans certains modes de réalisation, la pompe peut être incluse dans le dispositif. Dans certains de ces modes de réalisation, la pompe peut comprendre une vanne électromécanique en communication sous vide avec une source permettant d'établir le vide et un microprocesseur régulant la pression du vide dans le dispositif pour reproduire un cycle d'allaitement naturel.

Un mode de réalisation préféré de l'invention va maintenant être décrit à titre d'exemple uniquement et en référence aux Figures dans lesquelles :

### Brève description des figures

**Figure 1** représente une vue isométrique d'un premier mode de réalisation du dispositif selon l'appareil de la présente invention ;
**Figure 2** représente une vue isométrique du dispositif représenté dans la **Figure 1** connecté à un mode de réalisation d'une pompe manuelle ;
**Figure 3** représente une vue isométrique inversée du mode de réalisation de l'appareil représenté dans la **Figure 2** ;
**Figure 4** représente une vue isométrique du dispositif représenté dans la **Figure 1** connecté à un mode de réalisation d'une pompe électrique ;
**Figure 5** représente une vue isométrique inversée du mode de réalisation de l'appareil représenté dans la **Figure 4** ;
**Figure 6** représente une vue isométrique inversée du mode de réalisation du dispositif représenté dans la **Figure 1** ;
**Figure 7** représente une vue isométrique d'un mode de réalisation d'un récipient pour l'appareil selon la présente invention ;
**Figure 8** représente une vue isométrique explosée du mode de réalisation du récipient représenté dans la **Figure 7****,** avec un couvercle ;
**Figure 9** représente une vue isométrique explosée du mode de réalisation de dispositif représenté dans la **Figure 1** ; et
**Figure 10** représente une vue isométrique inversée du mode de réalisation du dispositif représenté dans la **Figure 1****.**

### Description détaillée des Figures

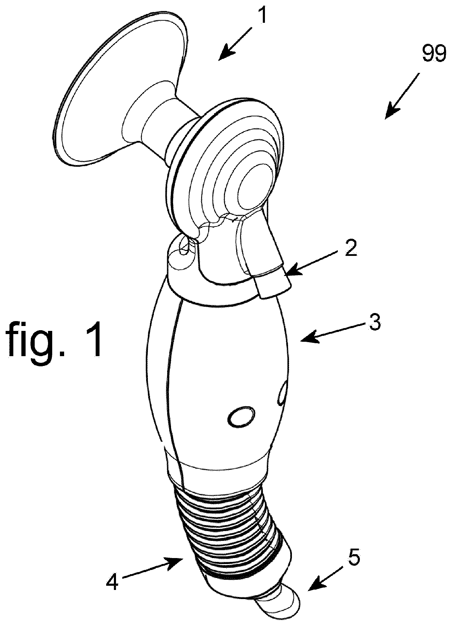

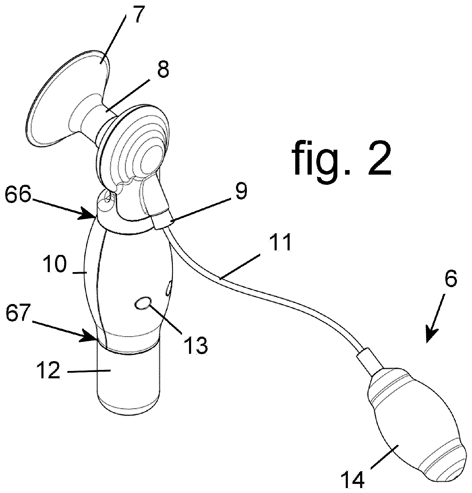

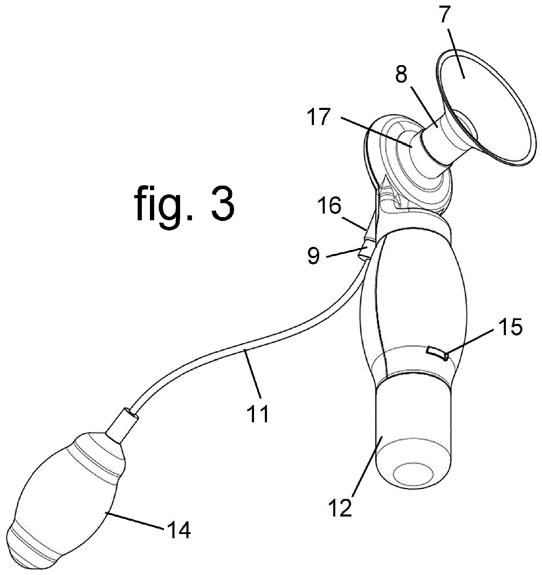

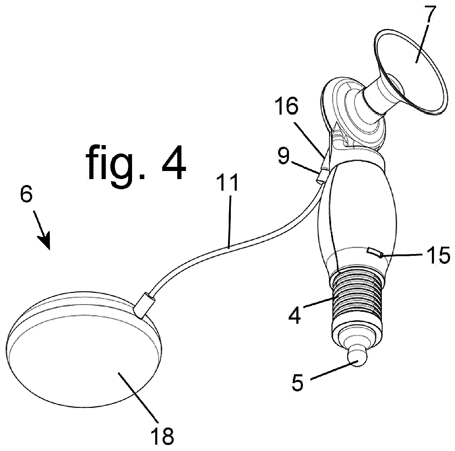

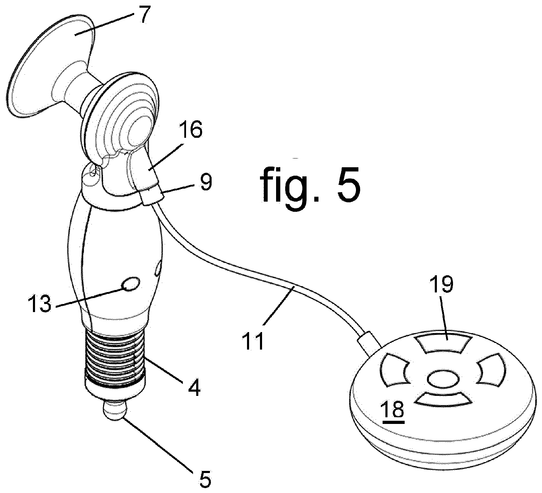

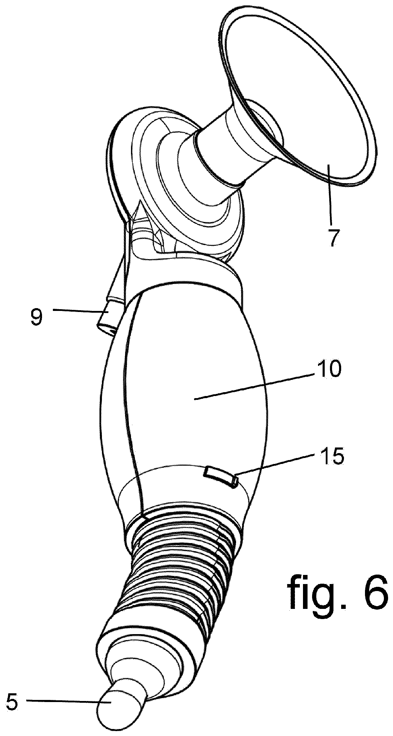

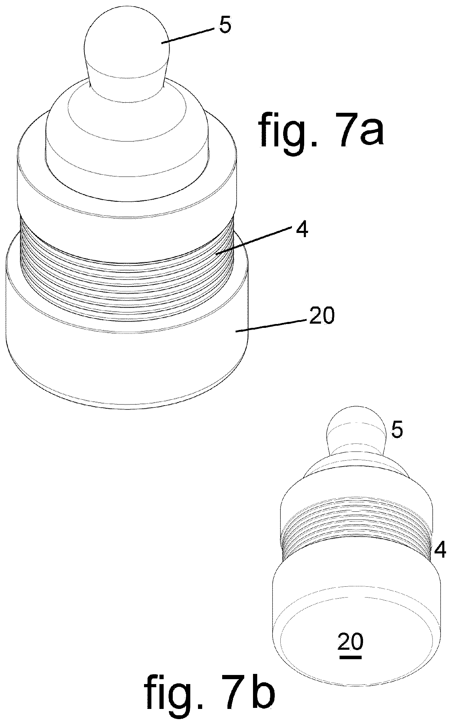

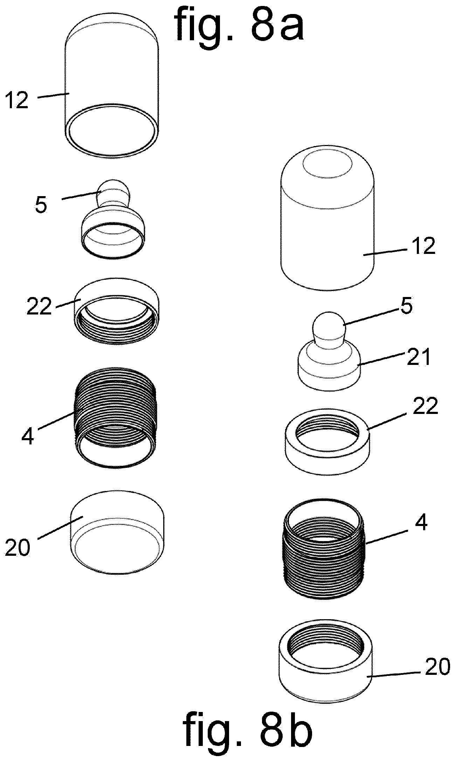

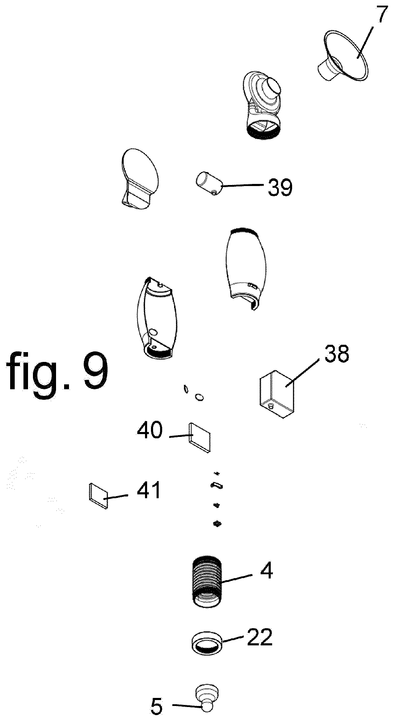

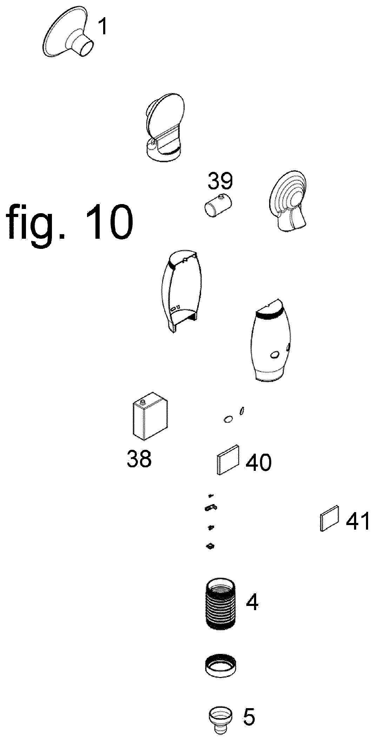

En référence aux **Figures 1 à 10****,** il est généralement représenté un appareil comprenant : un dispositif 99 avec un adaptateur mammaire 1 ; et une pompe 6 pour aspirer un fluide à partir de l'adaptateur mammaire 1 ; dans lequel le dispositif 99 comprend une tétine 5 et des moyens de mesure de fluide 3 pour mesurer le volume de fluide à l'intérieur du dispositif 99.

En référence particulière au mode de réalisation de l'appareil représenté, le mode de réalisation du dispositif 99 comprend un corps sensiblement allongé 10, s'étendant d'une première extrémité 66 comprenant un embout conique 7 formant l'adaptateur mammaire 1, à une seconde extrémité 67 connectée à une tétine 5.

L'embout 7 est composé d'un matériau flexible ou élastomère, agencé pour se déformer afin d'agir efficacement sous l'effet de la succion pour recouvrir, tenir ou serrer le sein ou le mamelon (non représenté) en utilisation, lorsque la pompe 6 est actionnée.

La tétine est située sur l'extrémité d'un bras flexible 4, qui se présente sous forme de soufflet en silicone permettant d'ajuster la tétine 5 à divers angles.

Le mode de réalisation illustré du dispositif comprend, également, un connecteur 2 pour la pompe 6, laquelle pompe est entièrement amovible. Le connecteur standard 2 est une bonde déformable 9 maintenue dans un collier 16 et permet l'insertion d'une extrémité d'un tube 11 de sorte que le mode de réalisation peut utiliser une pompe manuelle ou électrique, permettant à l'utilisateur de choisir son procédé préféré. La pompe électrique possède une série de commandes standard 19 permettant de régler la pression de pompage et des capacités d'enregistrement, de stockage et de sélection de données dans le but de pouvoir sauvegarder une pression de pompage préférée.

La pompe 6 sur les figures est constituée par deux moyens de pompage différents, à savoir un moyen de pompage manuel sous forme de bulbe déformable 14, et un moyen de pompage électrique 18.

Ceci fournit une pompe d'allaitement révolutionnaire qui permet au lait d'être pompé directement de l'utilisateur à son bébé.

En tant que moyens de mesure, le dispositif 99 comprend une électrovanne 39 et un débitmètre 38, qui fonctionnent ensemble pour contrôler l'écoulement du lait ou l'arrêter et indiquer à l'utilisateur la quantité de lait qui a traversé le dispositif.

Ces moyens de mesure sont alimentés par une batterie rechargeable qui peut être chargée à l'aide d'un port micro USB 15 (port de bus universel en série) qui est recouvert par un capuchon fixe en silicone déplaçable lors de l'utilisation du port USB.

Le dispositif comprend en outre un affichage visuel, qui est situé derrière le corps 10 du dispositif, lequel corps est formé de thermoplastiques translucides, de telle sorte que l'affichage visuel peut être lu à travers le corps.

Le mode de réalisation du dispositif comprend les éléments suivants :
- un débitmètre 38
- une électrovanne 39
- une batterie 40
- PCB 41 (carte de circuit imprimé)
- un corps extérieur 10
- un embout de type ventouse 7

Le goulot 8 est agencé pour s'enfiler et être repositionné de manière déformable dans le collier basal 17 à la première extrémité 66 du dispositif.

Le mode de réalisation comprend des boutons de commande de l'alimentation et des vannes 13 afin de contrôler si l'ouverture et la fermeture des vannes.

L'affichage peut également informer l'utilisateur lorsque les vannes sont ouvertes ou fermées.

Comme représenté sur les Figures, l'appareil est multipartite, comprenant un couvercle hygiénique 12 pour la tétine lorsqu'elle n'est pas utilisée.

La tétine fait partie d'un col en silicone 21, qui est maintenu en place sur le bras 4 par un collier 22, dans lequel le bras 4 est suffisamment rigide entre les soufflets compressibles pour se visser dans la seconde extrémité 67 du dispositif. Le bras peut ensuite être retiré du dispositif de sorte qu'un capuchon 20 puisse être vissé sur le bras 4 à la place de l'extrémité 67.

Ceci permet à la tétine 5 et au bras 4 de former une base à utiliser comme un récipient de type bouteille ou biberon pour alimenter le bébé, qui peut être placé dans le réfrigérateur après la collecte du lait, prêt à l'emploi.

Une fois le couvercle 12 retiré, la tétine 5 peut être étendue et dirigée vers le bébé pour une consommation facile et confortable, permettant également un stockage plus compact.

## Revendications

1. Appareil tire-lait comprenant : un dispositif (99) avec un adaptateur mammaire (1) et une pompe (6) pour aspirer le fluide à partir de l'adaptateur mammaire (1) ; dans lequel le dispositif comprend une tétine (5), ainsi que des moyens de mesure de fluide (3) pour mesurer le volume de fluide à l'intérieur du dispositif, **caractérisé en ce que** le dispositif comprend un bras articulé (4) supportant la tétine (5), permettant une variation d'angle rapport à l'adaptateur mammaire (1)

2. Appareil selon la revendication 1, **caractérisé en ce que** l'adaptateur mammaire est disposé sur un mécanisme de support s'étendant depuis le corps, permettant une variation du positionnement de l'adaptateur par rapport au corps.

3. Appareil selon la revendication 1, dans lequel la pompe (6) comprend un moyen de pompage électrique.

4. Appareil selon la revendication 1 ou la revendication 2 dans lequel la pompe (6) comprend un moyen détachable de pompage manuel.

5. Appareil selon l'une quelconque des revendications précédentes dans lequel l'adaptateur mammaire (1) comprend un embout souple (7).

6. Appareil selon l'une quelconque des revendications précédentes, dans lequel la tétine (5) peut être détachée du dispositif (99).

7. Appareil selon l'une quelconque des revendications précédentes comprenant un couvercle (12) pour la tétine (5).

8. Appareil selon l'une quelconque des revendications précédentes comprenant une base de biberon (20) pour la tétine (5).

9. Appareil selon l'une quelconque des revendications précédentes **caractérisé en ce qu'**il comprend un réservoir apte à effectuer un stockage temporaire de fluide pendant l'alimentation.

10. Appareil selon l'une quelconque des revendications précédentes dans lequel le dispositif comprend un affichage visuel.

11. Appareil selon l'une quelconque des revendications précédentes **caractérisé en ce qu'**il comprend des moyens de connexion sans fil aptes à fournir un affichage visuel à distance appartenant au groupe formé par Bluetooth, infrarouge et radiofréquence.

12. Appareil selon les revendications 1 à 11, **caractérisé en ce que** les moyens de mesure de fluide (3) sont aptes à mesurer des paramètres appartenant au groupe formé par : la température, et la vitesse d'écoulement.

13. Appareil selon les revendications 1 à 12, **caractérisé en ce que** les moyens de mesure de fluide (3) sont aptes à mesurer l'écoulement au niveau du groupe constitué par : à travers la tétine, à travers l'adaptateur mammaire.

14. Appareil selon les revendications 1 à 13, **caractérisé en ce que** les moyens de mesure de fluide (3) sont aptes à mesurer le volume dans le réservoir et/ou le volume de fluide à partir de l'adaptateur mammaire.

15. Appareil selon les revendications 1 à 14, **caractérisé en ce que** la pompe (6) comprend une vanne électromécanique en communication sous vide avec une source permettant d'établir le vide et un microprocesseur régulant ladite pression du vide dans le dispositif (99).

## Patentansprüche

1. Milchpumpengerät, das folgendes umfasst: eine Vorrichtung (99) mit einem Brustadapter (1) und einer Pumpe (6) zum Ansaugen der Flüssigkeit mit dem Brustadapter (1); wobei die Vorrichtung einen Sauger (5) sowie Mittel (3) zum Messen des Flüssigkeitsvolumens im Innern der Vorrichtung umfasst, **dadurch gekennzeichnet, dass** die Vorrichtung einen Gelenkarm (4) umfasst, der den Sauger (5) hält und eine Veränderung des Winkels relativ zum Brustadapter (1) ermöglicht.

2. Gerät nach Anspruch 1, **dadurch gekennzeichnet, dass** der Brustadapter auf einem sich vom Körper aus erstreckenden Stützmechanismus angeordnet ist, wodurch eine Veränderung der Positionierung des Adapters relativ zum Körper möglich ist.

3. Gerät nach Anspruch 1, in dem die Pumpe (6) ein elektrisches Pumpmittel umfasst.

4. Gerät nach Anspruch 1 oder 2, in dem die Pumpe (6) ein abnehmbares manuelles Pumpmittel umfasst.

5. Gerät nach einem der vorhergehenden Ansprüche, in dem der Brustadapter (1) einen flexiblen Aufsatz (7) umfasst.

6. Gerät nach einem der vorhergehenden Ansprüche, in dem der Sauger (5) von der Vorrichtung (99) abgenommen werden kann.

7. Gerät nach einem der vorhergehenden Ansprüche, das eine Abdeckung (12) für den Sauger (5) umfasst.

8. Gerät nach einem der vorhergehenden Ansprüche, das ein Flaschenunterteil (20) für den Sauger (5) umfasst.

9. Gerät nach einem der vorhergehenden Ansprüche, **dadurch gekennzeichnet, dass** es einen Vorratsbehälter umfasst, in dem während der Versorgung mit Milch vorübergehend Flüssigkeit bevorratet werden kann.

10. Gerät nach einem der vorhergehenden Ansprüche, in dem die Vorrichtung eine visuelle Anzeige umfasst.

11. Gerät nach einem der vorhergehenden Ansprüche, **dadurch gekennzeichnet, dass** es drahtlose Verbindungsmittel umfasst, die in der Lage sind, eine visuelle Fernanzeige zu liefern, wobei diese zur Gruppe der Bluetooth-, Infrarot- und Funkfrequenz-Verbindungen gehören.

12. Gerät nach den Ansprüchen 1 bis 11, **dadurch gekennzeichnet, dass** die Flüssigkeitsmessmittel (3) in der Lage sind, Parameter zu messen, die zur Gruppe bestehend aus Temperatur und Fließgeschwindigkeit gehören.

13. Gerät nach den Ansprüchen 1 bis 12, **dadurch gekennzeichnet, dass** die Flüssigkeitsmessmittel (3) in der Lage ist, den Durchfluss an einer Gruppe bestehend aus Kriterien wie "durch den Sauger", "durch den Brustadapter" zu messen.

14. Gerät nach den Ansprüchen 1 bis 13, **dadurch gekennzeichnet, dass** die Flüssigkeitsmessmittel (3) in der Lage sind, das Volumen im Vorratsbehälter und/oder das Volumen der Flüssigkeit am Brustadapter zu messen.

15. Gerät nach den Ansprüchen 1 bis 14, **dadurch gekennzeichnet, dass** die Pumpe (6) ein elektromechanisches Ventil umfasst, das in Vakuumverbindung mit einer Quelle zur Herstellung eines Vakuums steht, und einen Mikroprozessor zur Regelung des Vakuumdrucks in der Vorrichtung (99).

## Claims

1. Breast pump apparatus comprising: a device (99) with a breast adapter (1) and a pump (6) for sucking fluid from the breast adapter (1); wherein the device comprises a nipple (5), as well as fluid measuring means (3) for measuring the volume of fluid inside the device, **characterised in that** the device comprises an articulated arm (4) supporting the nipple (5), allowing a variation in angle with respect to the breast adapter (1).

2. Apparatus according to claim 1, **characterised in that** the breast adapter is arranged on a support mechanism extending from the body, allowing variation in the positioning of the adapter relative to the body.

3. Apparatus according to claim 1, wherein the pump (6) comprises an electrical pumping means.

4. Apparatus according to claim 1 or claim 2, wherein the pump (6) comprises a detachable manual pumping means.

5. Apparatus according to any of the foregoing claims, wherein the breast adapter (1) comprises a soft tip (7) .

6. Apparatus according to any of the foregoing claims, wherein the nipple (5) can be detached from the device (99).

7. Apparatus according to any of the foregoing claims, comprising a cover (12) for the nipple (5).

8. Apparatus according to any of the foregoing claims, comprising a baby's bottle base (20) for the nipple (5) .

9. Apparatus according to any of the preceding claims, **characterised in that** it comprises a reservoir capable of effecting a temporary storage of fluid during feeding.

10. Apparatus according to any of the foregoing claims, wherein the apparatus includes a visual display.

11. Apparatus according to any of the preceding claims, **characterised in that** it comprises wireless connection means capable of providing a remote visual display belonging to the group formed by Bluetooth, infrared and radio frequency.

12. Apparatus according to claims 1 to 11, **characterised in that** the fluid measuring means (3) are capable of measuring parameters belonging to the group formed by: temperature and flow velocity.

13. Apparatus according to claims 1 to 12, **characterised in that** the fluid measuring means (3) are capable of measuring the flow at the group consisting of: through the nipple, through the breast adapter.

14. Apparatus according to claims 1 to 13, **characterised in that** the fluid measuring means (3) are adapted to measure the volume in the reservoir and/or the volume of fluid from the breast adapter.

15. Apparatus according to claims 1 to 14, **characterised in that** the pump (6) comprises an electromechanical valve in vacuum communication with a source for establishing a vacuum and a microprocessor regulating said vacuum pressure in the device (99).
